# EUROPÄISCHE PATENTSCHRIFT

(11) **EP 1 290 035 B1**
(45) Veröffentlichungstag und Bekanntmachung des Hinweises auf die Patenterteilung: **11.02.2004**
(21) Anmeldenummer: 01940413.6
(22) Anmeldetag: 08.05.2001
(51) Int. Cl.: C08B 37/00, A61K 49/00

(54) **FLUORESCEINISOTHIOCYANAT-(FITC)-SINISTRIN, SEINE HERSTELLUNG UND VERWENDUNG**
FLUORESCEIN ISOTHIOCYANATE (FITC) SINISTRIN, ITS PRODUCTION AND USE
SINISTRINE D'ISOTHIOCYANATE DE FLUORESCEINE (FITC), FABRICATION ET UTILISATION

(30) Priorität: 11.05.2000 DE 10023051
(43) Veröffentlichungstag der Anmeldung: 12.03.2003
(73) Patentinhaber: Roche Diagnostics GmbH, 68305 Mannheim (DE); F.HOFFMANN-LA ROCHE AG, 4070 Basel (CH)
(72) Erfinder: HEIN, Heinz-Michael, 64295 Darmstadt (DE); KRAEMER, Uwe, 68549 Ilvesheim (DE); REITER, Rudolf, 31275 Lehrte (DE); GRETZ, Norbert, 68259 Mannheim (DE); DEUS, Carsten, 69168 Wiesloch (DE)
(86) Internationale Anmeldenummer: PCT/EP2001/005172
(87) Internationale Veröffentlichungsnummer: WO 2001/085799

(56) Entgegenhaltungen:
- DE-A- 4 101 910

## Beschreibung

Die Erfindung betrifft eine neue chemische Verbindung, die als Markersubstanz in der Nierendiagnostik eingesetzt werden kann, ihre Herstellung und Verwendung sowie ein sie enthaltendes Nierendiagnostikum.

In der Nierendiagnostik, dort insbesondere bei der Bestimmung der glomerulären Filtrationsrate (GFR) zur Nierenfunktionsprüfung, werden unter anderem Fructane als Markersubstanzen eingesetzt. Fructane, die auch als Polyfructosane bezeichnet werden, sind Oligo- und Polysaccharide, die aus geraden oder verzweigten Fructoseketten aufgebaut sind, welche auf ein Grundmolekül Saccharose aufgepfropft sind. Je nach Grad der Verzweigung der Fructoseketten und je nach Grad der Polymerisation können unterschiedliche Fructane unterschiedliche physikalische Eigenschaften aufweisen, beispielsweise unterschiedliche Wasserlöslichkeiten. Viele Fructane kommen in Pflanzen als Reservekohlenhydrate vor, beispielsweise in den unterirdischen Teilen von Compositen, Campanulaceen, Gräsern und Liliaceen.

In der Nierenfunktionsprüfung werden insbesondere die Fructane Inulin und Sinistrin als Markersubstanzen eingesetzt. Inulin und Sinistrin sind aus jeweils ca. 10 bis 40 Fructoseeinheiten aufgebaut und weisen dementsprechend Molekulargewichte von ca. 1600 bis ca. 6500 auf. Sowohl Inulin als auch Sinistrin werden nach parenteraler Applikation weder durch den Stoffwechsel verändert noch im Organismus gespeichert, sondern durch die Nierenglomeruli ausfiltriert und in den Tubuli nicht wieder rückresorbiert.

Zur Beurteilung der Nierenfunktion wird üblicherweise der zeitliche Verlauf der Markersustanzkonzentration im Blut nach parenteraler Gabe einer bestimmten Dosis der Markersubstanz bestimmt. Die Bestimmung der Konzentration der Markersubstanz im Blut kann beispielsweise mittels enzymatischer Methoden erfolgen (vgl. z. B. H.F. Kuehnle et al., Fully enzymatic inulin determination in small volume samples without deproteinization, Nephron 62 (1992) 104 - 107). Im Fall von Inulin als Markersubstanz wird unter anderem auch die Möglichkeit beschrieben, mit einem Fluoreszenzmarker versehenes Inulin, beispielsweise Fluoresceinisothiocyanat-markiertes Inulin (FITC-Inulin), zu verwenden und die Konzentration der Markersubstanz mittels Fluoreszenzmessung zu bestimmen (vgl. z. B. M. Sohtell *et al*., FITC-inulin as a kidney tubule marker in the rat, Acta Physiol. Scand. 119 (1983) 313-316; J. N. Lorenz & E. Gruenstein, A simple, nonradioactive method for evaluating single-nephron filtration rate using FITC-inulin, Am. J. Physiol. 276 (Renal Physiol. 45) (1999) F172-F177).

Inulin und FITC-Inulin haben für die klinische Alltagsroutine den Nachteil, daß sie in Wasser nur sehr gering löslich sind und in wässrigen Zubereitungen bei der Lagerung auskristallisieren. Vor der Applikation müssen die inulinhaltigen Zubereitungen deshalb in der Regel erwärmt werden, um das Inulin oder FITC-Inulin wieder in Lösung zu bringen. Durch diese Maßnahme wird das Inulin jedoch je nach Dauer des Erhitzens hydrolytisch angegriffen und teilweise bis zur Fructose abgebaut. Zudem verbleiben bei unvollständigem Auflösen Reste von nicht gelösten Inulinteilchen in der Zubereitung, die nur schwer zu erkennen sind und die nach einer Injektion schwere Kreislaufkomplikationen zur Folge haben können. Die geringe Löslichkeit von Inulin oder FITC-Inulin führt dazu, daß eine definierte Konzentration der Markersubstanz in einer Injektionslösung nur schwer zu erreichen ist. Außerdem hat die Anwendung von Inulin oder FITC-Inulin nach der Injektion in das Versuchstier einen transienten Blutdruckabfall zur Folge. Diese Kreislaufreaktion dauert in günstigen Fällen 5 Minuten. Insbesondere wird durch den Kreislaufeinbruch gerade die zu bestimmende Nierenfunktion gestört.

Sinistrin ist wie Inulin ein Fructan und kann durch Extraktion aus fructanhaltigen Pflanzenteilen gewonnen werden (vgl. z. B. EP-B 0 568 574). Die Verwendung von Sinistrin als Markersubstanz erfordert jedoch verhältnismäßig hohe Sinistrinkonzentrationen in den entsprechenden Zubereitungen, die im Bereich von 100 mg je kg Körpergewicht des zu untersuchenden Individuums liegen, da Sinistrin selbst nur in Blutproben bestimmt werden kann und die hierfür zur Verfügung stehenden Analysenmethoden verhältnismäßig unempfindlich sind. Zudem ist der Nachweis von Sinistrin nur mit einer mehrstufigen enzymatischen Reaktion möglich, bei der zunächst nach Entfernen der endogenen Glucose das Sinistrin in Glucose umgewandelt und die so erhaltene Glucose als Maß für das Sinistrin bestimmt wird. Erfahrungsgemäß sind solche mehrstufigen Reaktionen aufwendig und oft mit einem nicht unerheblichen Fehler behaftet.

Aufgabe der vorliegenden Erfindung ist es daher, die Nachteile des Standes der Technik zu beseitigen. Insbesondere ist es die Aufgabe der vorliegenden Erfindung, eine Substanz bereitzustellen, die als Markersubstanz in der Nierenfunktionsprüfung eingesetzt werden kann und die gegenüber den im Stand der Technik bekannten Markersubstanzen, insbesondere Inulin, FITC-Inulin und Sinistrin, Vorteile aufweist.

Die Aufgabe wird durch den Gegenstand der Erfindung, wie er in den Patentansprüchen angegeben ist, gelöst.

Gegenstand der Erfindung ist mit Fluoresceinisothiocyanat gelabeltes Sinistrin, welches im Folgenden als FITC-Sinistrin bezeichnet werden soll.

Das FITC-Sinistrin der vorliegenden Erfindung ist erhältlich durch Umsetzung von Sinistrin mit Fluoresceinisothiocyanat (FITC), wobei zunächst das Sinistrin in einem geeigneten Lösungsmittel, beispielsweise Dimethylformamid (DMF), mit Natriumhydrid (NaH) zur Reaktion gebracht wird und anschließend FITC zur Reaktionsmischung gegeben wird. Das Produkt, FITC-Sinistrin, kann nach an sich bekannten Methoden, beispielsweise durch Versetzen mit wäßriger Ammoniumchloridlösung (NH₄Cl), anschließende Extraktion mit Diethylether und Entfernen des Lösungsmittels, als Feststoff isoliert und gegebenenfalls durch Umkristallisieren und/oder Gelfiltration gereinigt werden. Eine bevorzugte Darstellungsvorschrift für FITC-Sinistrin ist in Beispiel 1 angegeben.

Ein weiterer Gegenstand der Erfindung ist das oben beschriebene Herstellverfahren für FITC-Sinistrin.

Weiterhin ist Gegenstand der Erfindung die Verwendung von FITC-Sinistrin als Bestandteil einer diagnostischen Zubereitung, die insbesondere für die Nierendiagnostik geeignet ist, sowie ein Diagnostikum, insbesondere eine diagnostische Zubereitung, das FITC-Sinistrin enthält.

Das FITC-Sinistrin der vorliegenden Erfindung wird bevorzugt als Bestandteil einer parenteral zu verabreichenden Zubereitung für die Nierenfunktionsprüfung verwendet. Zur Herstellung des Diagnostikums wird FITC-Sinistrin in *aqua ad inj.* (Wasser für Injektionszwecke gemäß DAB 10) oder physiologischer Kochsalzlösung (isotonische Natriumchloridlösung) gelöst. Die Konzentration des FITC-Sinistrin in der diagnostischen Zubereitung liegt im Bereich von 25 bis 125 mg/ml. Neben FITC-Sinistrin kann das parenteral zu applizierende Diagnostikum auch noch physiologisch verträgliche Puffersubstanzen enthalten.

Die Anwesenheit der Fluoresceinisothiocyanat-Gruppe in FITC-Sinistrin ermöglicht die Bestimmung von FITC-Sinistrin anhand von Fluoreszenzmessungen. Diese können in vitro durchgeführt werden, beispielsweise in Blutproben. Für die Fluoreszenzmessung von FITC-Sinistrin, in beispielsweise Blutproben, ist keine enzymatische Vorbehandlung der Blutprobe notwendig. Zudem bietet die Fluoreszenzmessung den Vorteil hoher Empfindlichkeit und Schnelligkeit der Messung. Die Messung kann mit üblichen Standardgeräten durchgeführt werden. Der Einsatz des erfindungsgemäßen FITC-Sinistrins als Markersubstanz in der Nierendiagnostik erlaubt auch nicht-invasive Nachweismethoden für FITC-Sinistrin. Nicht-invasive Nachweismethoden sind im hier benutzten Sprachgebrauch Methoden, die den Nachweis einer Substanz, hier von FITC-Sinistrin, in Gewebe oder Körperflüssigkeiten ohne vorherige Probennahme, beispielsweise durch Blutabnahme nach einer Venenpunktion oder durch Gewinnung von Kapillarblut aus der Fingerbeere oder dem Ohrläppchen, erlauben.

Vorzugsweise wird als nicht-invasives Verfahren zur Bestimmung von FITC-Sinistrin im Gewebe oder in Körperflüssigkeiten ein Fluoreszenzmessverfahren eingesetzt, bei dem Licht zur Anregung der Fluoreszenz in die Haut des zu untersuchenden Individuums eingestrahlt wird und das aus der Haut austretende Fluoreszenzlicht detektiert wird. Vorteilhafterweise kann dies mit Hilfe eines nicht-invasiven Messkopfes geschehen, bei dem eine Lichtquelle, beispielsweise ein im UV-Bereich emittierender Laser, über eine Glasfaseroptik die Haut beleuchtet und die darin enthaltenen FITC-Sinistrinmoleküle zur Fluoreszenz anregt. Das Fluoreszenzlicht wird mit einer Glasfaseroptik aufgenommen und mit einem entsprechenden Detektor, beispielsweise einem CCD-Spektrographen, gemessen. Die Lichtquelle und/oder der Detektor kann dabei in den Messkopf integriert oder außerhalb des Meßkopfes angeordnet sein. Der Meßkopf wird mit einem transparenten Kleber, beispielsweise einer transparenten Klebefolie, auf die Haut des zu untersuchenden Individuums aufgeklebt und verbleibt dort für die gesamte Meßzeit.

Da die Bestimmung von FITC-Sinistrin mit Hilfe empfindlicher Fluoreszenzmessungen möglich ist, kann die FITC-Sinistrin-Menge, die dem zu untersuchenden Individuum verabreicht wird, deutlich niedriger sein, als dies mit (underivatisiertem) Sinistrin der Fall ist. Während für Sinistrin Dosen von 100 mg Substanz je kg Körpergewicht des zu untersuchenden Individuums notwendig sind, kann FITC-Sinistrin ausreichend sensitiv bereits bei Dosen von 5 bis 50 mg, vorzugsweise sogar schon bei Dosen von 5 bis 20 mg Substanz je kg Körpergewicht des zu untersuchenden Individuums nachgewiesen werden. Durch die niedrige Dosierung wird eine Belastung des zu untersuchenden Organismus' im Vergleich zu Sinistrin deutlich vermindert.

Zudem ist eine nicht-invasive Detektion von FITC-Sinistrin möglich. Dies trägt ebenfalls zur Reduzierung der körperlichen Beeinträchtigung des zu untersuchenden Individuums bei, da keine Blutproben für die Untersuchung und Bestimmung von FITC-Sinistrin genommen werden müssen.

Die nicht-invasive Messung des FITC-Sinistringehalts kann kontinuierlich über einen längeren Zeitraum, beispielsweise über die klinisch relevante Meßzeit von 180 min für die Nierenfunktionskontrolle (gFR), erfolgen. Dies trägt zu einer präzisen Diagnostik bei.

Bislang wurden bei der Verwendung von FITC-Sinistrin als Markersubstanz für die Nierenfunktionsprüfung keine unerwünschten Kreislaufreaktionen bei den untersuchten Individuen festgestellt. Die glomeruläre Filtrationsrate kann somit ohne sekundären Einfluss auf die Niere bestimmt werden. Dies ist ein deutlicher Vorteil gegenüber der bekannten Verwendung von FITC-Inulin oder Inulin.

Die vorliegende Erfindung wird durch die nachfolgenden Beispiele näher erläutert:

### Beispiel 1:

### Herstellung von Fluoresceinisothiocyanat-Sinstrin

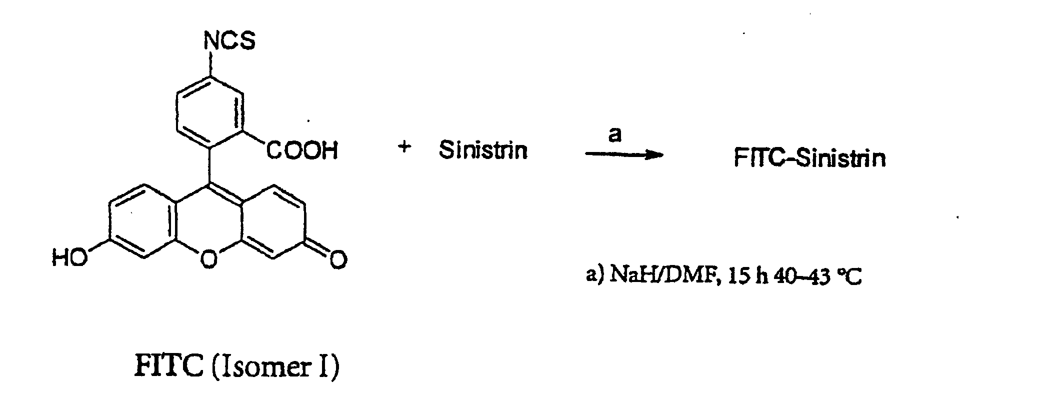

Sinistrin (500 mg, 3,1 mmol, Fresenius Kabi, Linz, Österreich) wurde unter N₂-Atmosphäre zu 17 ml trockenem Dimethylformamid (DMF) gegeben und 15 min bei 40-43°C gerührt. Es wurde im Eisbad abgekühlt und mit Natriumhydrid (500 mg einer 60-prozentigen Suspension in Öl, 12,5 mmol, Fluka, Buchs, Schweiz) versetzt. Es wurde 5 min bei Raumtemperatur und 30 min bei 40-45°C gerührt, wobei die Reaktionsmischung zunehmend viskos wurde, jedoch noch rührbar blieb. Fluoresceinisothiocyanat (FITC, 350 mg, 0,9 mmol, Sigma, Isomer I) wurde als Feststoff zugegeben. Dabei sank sofort die Viskosität der Reaktionsmischung. Nach 18 h Rühren bei 40-45°C wurde auf 0°C gekühlt und vorsichtig mit einer Lösung von Ammoniumchlorid (NH₄Cl, 696 mg, 13 mmol) in 10 ml Wasser versetzt. Nach der Zugabe von weiteren 20 ml Wasser wurde die trübe Lösung zum Entfernen des Weißöls zweimal mit Diethylether extrahiert. Das Lösungsmittel wurde am Rotationsverdampfer bei einer Badtemperatur von unter 40°C weitestgehend entfernt und der Rückstand im Hochvakuum getrocknet. Der Rückstand wurde in 30 ml einer 1:1-Mischung von Ethanol und Wasser aufgenommen und erst mit Ethanol, dann mit Aceton bis zu einem Gesamtvolumen von 1000 ml gefällt. Der Niederschlag wurde sedimentiert und abzentrifugiert.

Nach einer Gelfiltration (BioRad Biogel, P-2 extra fine, Säule 2,5 * 35 cm, Eluens VE-Wasser) konnte durch erneute Fällung 340 mg FITC-Sinistrin als gelbes Pulver erhalten werden.

Bezogen auf 1 mol Sinistrin enthielt das Produkt ca. 0,14 mol FITC.

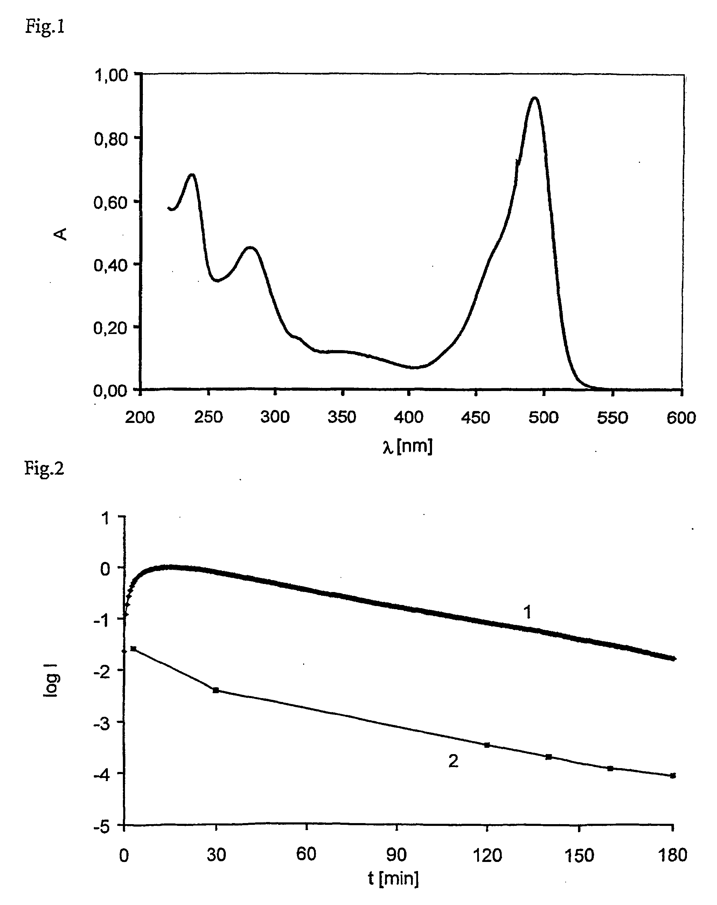

Figur 1 zeigt ein UV/VIS-Spektrum von FITC-Sinistrin, bei dem die Absorption A gegen die Wellenlänge in λ nm aufgetragen ist.

### Beispiel 2

### Nicht-invasive Messung von FITC-Sinistrin im Tierversuch

### a) Einsatz des nicht-invasiven Meßkopfes:

Der nicht-invasive Meßkopf hatte die Aufgabe, Licht zur Anregung der Fluoreszenz in die Haut einzustrahlen und Fluoreszenzlicht aus der Haut aufzunehmen. Der Meßkopf war als faseroptischer Messkopf gestaltet, bei dem eine externe Lichtquelle (UV-Laser) über eine Glasfaser die Haut beleuchtete und die darin enthaltenen FITC-Sinistrin-Moleküle anregte. Das Fluoreszenzlicht (520 nm Wellenlänge) wurde wiederum mit Glasfasern aufgenommen und in einem externen Detektor (CCD-Spektrograph) gemessen.

Der Meßkopf wurde mit einer transparenten Klebefolie auf die Haut des Versuchstieres aufgeklebt und verblieb dort für die gesamte Meßzeit.

### b) Durchführung der Tierexperimente:

Das Versuchstier (Kaninchen) wurde fachgerecht anästhesiert und mit einem zentralarteriellen Katheter versehen. Der Katheter diente lediglich zum Überwachen des arteriellen Blutdrucks und zur Entnahme von Referenzblutproben.

Der nicht-invasive Meßkopf wurde im Bereich des Brustkorbes des Versuchstieres auf einer rasierten Hautstelle aufgeklebt. Die Testsubstanz FITC-Sinistrin wurde in einer Standarddosis von 30 mg/kg intravenös appliziert. Die gesamte, klinisch relevante Messzeit für die Nierenfunktionsprüfung (gFR) betrug 180 min.

### c) Ergebnis der Tierexperimente:

In Figur 2 ist ein Vergleich zwischen einer nicht-invasiven, durch Fluoreszenzmessung von FITC-Sinistrin erhaltenen Clearance-Kurve (1) mit einer FITC-Clearance-Kurve (2), deren Werte durch Fluoreszenzmessung in Blutproben des Versuchstieres gewonnen wurden, dargestellt. Dabei ist die normierte Signalintensität I logarithmisch gegen die Zeit t in min aufgetragen.

Die Übereinstimmung der Abfalldynamik der beiden Kurven, die sich auch durch die ähnlichen Halbwertszeiten für die Clearance ausdrückt, zeigt die Gleichwertigkeit der beiden Methoden. Die Halbwertszeit für die nicht-invasive Methode betrug in diesem Beispiel 38 min; für die invasive Referenzmethode wurden 43 min gefunden.

### Beispiel 3

### Vergleich der invasiven mit der nicht-invasiven Messmethode für Sinistrin und Inulin

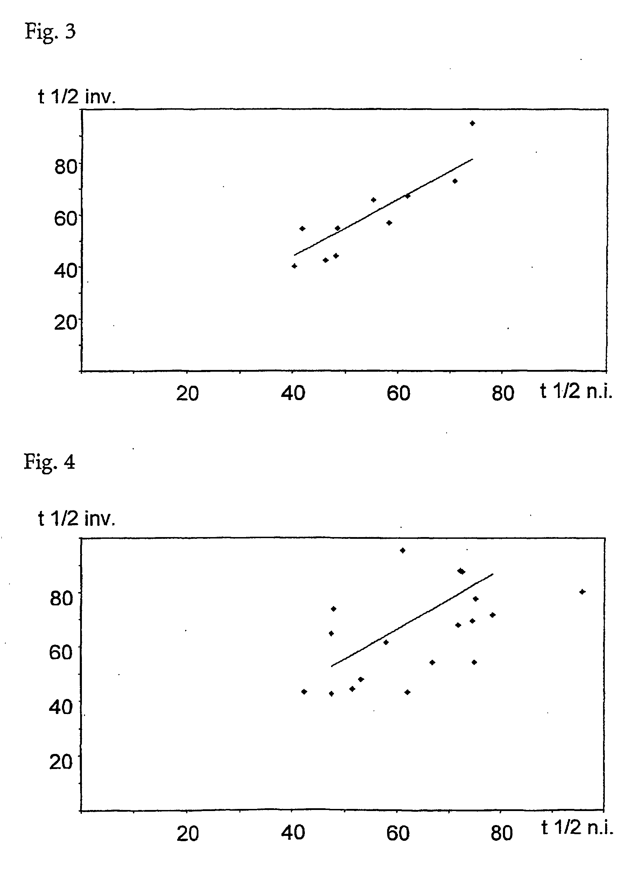

Das Tierexperiment aus Beispiel 2 wurde für FITC-Sinistrin 10 mal in identischer Weise wiederholt. Die Halbwertszeiten (in Minuten) für die Clearancekurven, die aus den invasiven Messungen gewonnen wurden (t 1/2 inv.), wurden gegen die aus den nicht-invasiven Messungen erhaltenen Halbwertszeiten (t 1/2 n.i.) aufgetragen. Die Ergebnisse sind in Figur 3 dargestellt. Der Korrelationskoeffizient betrug 89,9 %.

Zum Vergleich wurde das Tierexperiment aus Beispiel 2 für FITC-Inulin (erhältlich bei Sigma (Aldrich)) 20 mal in identischer Weise wiederholt. Die Halbwertszeiten (in Minuten) für die Clearancekurven, die aus den invasiven Messungen gewonnen wurden (t 1/2 inv.), wurden gegen die aus den nicht-invasiven Messungen erhaltenen Halbwertszeiten (t 1/2 n.i.) aufgetragen. Die Ergebnisse sind in Figur 4 dargestellt. Der Korrelationskoeffizient betrug in diesem Fall nur 49,2 %.

Während bei der Verwendung des erfindungsgemäßen FITC-Sinistrins eine sehr gute Korrelation zwischen invasiver und nicht-invasiver Messung gefunden wurde, ist für FITC-Inulin praktisch keine Korrelation zu erkennen. Möglicherweise ist die geringe Übereinstimmung im Fall von FITC-Inulin darauf zurückzuführen, dass durch FITC-Inulin die Physiologie des Versuchstieres so gestört wird, dass keine vernünftige Messung möglich ist. Mit dem erfindungsgemäßen FITC-Sinistrin tritt dieses Problem nicht auf.

### Beispiel 4

### Unterschiedliche Anreicherung von FITC-Sinistrin und FITC-Inulin in Organen von Versuchstieren

Einer Ratte wurden im Abstand von 2 Tagen intravenös jeweils 30 mg FITC-Sinistrin per kg Körpergewicht verabreicht. 4 Stunden nach der letzten Gabe von FITC-Sinistrin wurde das Tier getötet und Organschnitte von Lunge, Leber und Nieren des Versuchstiers angefertigt. Die Schnitte wurden mittels Fluoreszenzmessung auf Anwesenheit von FITC-Sinistrin untersucht. In keinem der genannten Organe konnte Fluoreszenz über dem Maß der Eigenfluoreszenz festgestellt werden. Alle Organe waren folglich frei von FITC-Sinistrin.

Ein identischer Versuch wurde mit FITC-Inulin durchgeführt. Hier ist in den genannten Organen Leber, Lunge und Nieren erhebliche Fluoreszenz zu beobachten, die auf Anwesenheit von FITC-Inulin zurückzuführen ist.

In der Leber wurde FITC-Inulin insbesondere intrazellulär vorgefunden. Dies hat eine irreversible Zellschädugung der Zelle (Zelltod) zur Folge und kann langfristig zur Schädigung des Organs führen. In der Lunge wurde FITC-Inulin korpuskulär angetroffen, was zu micro-Stenosen und in der Folge zur Embolie führt.

Auch bei Ratten, die bereits 6 Minuten nach der Injektion getötet wurden und sofort mit Kochsalzlösung gespült wurden, um alles Blut etc. aus dem Tier zu entfernen, ist beobachtbar, dass bei der Verwendung von FITC-Inulin eine Anreicherung dieser Substanz in Leber, Lunge und Nieren stattfindet. In diesem Versuch wurden also Anteile von FITC-Inulin gefunden, die sich sehr schnell in den Organen festsetzen. Bei der Verabreichung von FITC-Sinistrin war hingegen in keinem der oben genannten Organe Fluoreszenz zu finden.

### Beispiel 5

### Nachweis von FITC-Sinistrin mittels Teststreifen

Auf eine mittels Corona-Behandlung vorbehandelte, 125 µm dicke transparente Pokalonfolie wurde wie in EP-A 0 821 234 beschrieben eine zweilagigige Beschichtung mit den nachfolgend beschriebenen Beschichtungsmassen 1 und 2 aufgebracht. Die Schichten wurden in einer Dikke von jeweils 75 µm nacheinander (zuerst Beschichtungsmasse 1) bei einer Geschwindigkeit von 1 m/min aufgerakelt, wobei nach jeder Beschichtung für 30 min bei 50 °C getrocknet wurde. Die so erhaltenen Filme wurden in 6 mm * 6 mm große Stücke geschnitten und mittels eines beidseitig klebenden Klebebandes über ein kreisrundes Loch von 4 mm Durchmesser auf eine 100 mm lange, 6 mm breite und 1 mm dicke Polyesterfolie aufgeklebt, die im Wesentlichen der einfacheren Handhabung des Teststreifens diente. Dabei wurde die Pokalonfolie des beschichteten Films auf das Klebeband aufgelegt, so dass die Filmschicht aus Beschichtungsmasse 2 für die Probenaufgabe offen blieb.. Das Klebeband enthielt an der Stelle, an der die Polyesterfolie ein Loch aufwies, ebenfalls ein Loch mit 4 mm Durchmesser.

Auf die so hergestellten Teststreifen wurden 10 µl einer Probenlösung aufgegeben (auf die offene Seite des zweilagigen Films) und von der Unterseite (Pokalonfolie) mittels eines herkömmlichen Fluoreszenzdetektionsgeräts gemessen. Die gemessene Fluoreszenz bei 520 nm korrelierte dabei hervorragend mit der Menge an FITC-Sinistrin in der Probe.

| Beschichtungsmasse 1 (Mengenangaben in g je 100 g fertige Masse) | |
|---|---|
| Keltrol (1,4 %ig in Wasser) | 34,116 |
| Transpafill-Lösung (21,2 %ig in Wasser) | 41,069 |
| Propiofan 70 D | 4,347 |
| PVP-Lösung (14,117 g Wasser + 0,290 g Mega 8 + 0,041 Geropon T77) | 14,449 |
| Hexanol | 0,221 |
| Methoxypropanol | 5,824 |

| Beschichtungsmasse 2 (Mengenangaben in g je 100 g fertige Masse) | |
|---|---|
| Gantrez S97 (4 %ig in Wasser; mit 16 %iger NaOH auf pH 7 eingestellt) | 49,039 |
| TiO₂-Aufschlämmung (50 %ig in Wasser) | 38,871 |
| Propiofan 70 D | 3,880 |
| PVP-Lösung (7,909 g Wasser + 0,390 g Mega 8 + 0,040 g Geropon T77) | 8,339 |
| Hexanol | 0,215 |
| Methoxypropanol | 5,676 |

Allgemein ist für den FITC-Sinistrin-Nachweis mittels Teststreifen (oder auch nicht-Streifenförmigen Testelementen, beispielsweise im wesentlichen quadratische Plättchen) zu beachten, dass vorzugsweise eine saugfähige Matrix, beispielsweise aus Zellulose oder - wie im obigen Beispiel - aus einem saugfähigen Polymer oder Polymergemisch, vorhanden sein sollte. Die Filmmatrix kann ein oder mehrschichtig sein, z. B. aufgebaut als Polymer plus organischem Anteil. Bevorzugt enthalten die Filme keine herkömmlichen Nachweisenzyme und - reagenzien (wie dies bei herkömmlichen Teststreifen der Fall ist), da lediglich die Fluoreszenz der Probe gemessen werden soll. Wichtig ist insofern, dass die Grundsubstanzen für die Fertigung der Testelemete zum FITC-Sinistrinnachweis mittels Fluoreszenz keine oder möglichst wenig Eigenfluoreszenz zeigen.

Da die Fluoreszenz von der Proteinkonzentration in der Probe abhängig ist, kann es von Vorteil sein, auf einem Teststreifen eine Fluoreszenzmessung zur Bestimmung von FITC-Sinistrin zusammen mit einer Proteinmessung, wie sie beispielsweise aus Urinteststreifen bekannt ist, zu kombinieren. Gegebenenfalls sollte alternativ oder zusätzlich auf Hämoglobin als Ausschlußkriterium getestet werden, da hämolytische Proben verworfen werden müssen.
Auch die Hämoglobinbestimmmung mittels Teststreifen ist bekannt, beispielsweise aus Urinteststreifen. Vorzugsweise erfolgt die Bestimmung der genannten Parameter mittels Teststreifen, die mehrere, für den jeweiligen Parameter spezifische Testfelder aufweisen.

## Patentansprüche

1. Verfahren zur Herstellung von Fluoresceinisothiocyanat-Sinistrin (FITC-Sinistrin), wobei
i) zunächst das Sinistrin in einem geeigneten Lösungsmittel mit Natriumhydrid zur Reaktion gebracht wird;
ii) anschließend FITC zur Reaktionsmischung gegeben wird; und
iii) das FITC-Sinistrin als Feststoff isoliert wird.

2. Verfahren gemäß Anspruch 1, wobei das Lösungsmittel in Schritt i) trockenes Dimethylformamid ist.

3. Verfahren gemäß Anspruch 1 oder 2, wobei Schritt iii) das Versetzten der Reaktionsmischung mit wäßriger Ammoniumchloridlösung, die anschließende Extraktion mit Diethylether und das Entfernen des Lösungsmittels umfaßt.

4. Verfahren gemäß Anspruch 3, wobei
iv) der Feststoff aus Schritt iii) durch Umkristallisieren und/oder Gelfiltration gereinigt wird.

5. Fluoresceinisothiocyanat-Sinistrin (FITC-Sinistrin) erhältlich durch ein Verfahren gemäß einem der Ansprüche 1 bis 4.

6. Verwendung von Fluoresceinisothiocyanat-Sinistrin (FITC-Sinistrin) gemäß Anspruch 5 als Bestandteil einer diagnostischen Zubereitung für die Nierendiagnostik.

7. Diagnostische Zubereitung enthaltend Fluoresceinisothiocyanat-Sinistrin (FITC-Sinistrin).

8. Nicht-invasives Verfahren zur Messung der Nierenfunktion umfassend das Einstrahlen von Licht auf die Haut eines zu untersuchenden Individuums und das Erfassen des Fluoreszenzlichts aus der Haut, welches durch Fluoresceinisothiocyanat-Sinistrin (FITC-Sinistrin) gemäß Anspruch 5 oder die diagnostische Zubereitung gemäß Anspruch 7 erzeugt wird.

## Claims

1. Method for producing fluorescein isothiocyanate-sinistrin (FITC-sinistrin), wherein
i) the sinistrin is firstly reacted with sodium hydride in a suitable solvent;
ii) FITC is subsequently added to the reaction mixture and
iii) the FITC-sinistrin is isolated as a solid.

2. Method as claimed in claim 1, wherein the solvent in step i) is anhydrous dimethylformamide.

3. Method as claimed in claim 1 or 2, wherein step iii) comprises adding aqueous ammonium chloride solution to the reaction mixture, subsequent extraction with diethyl ether and removal of the solvent.

4. Method as claimed in claim 3, wherein
iv) the solid from step iii) is purified by recrystallization and/or gel filtration.

5. Fluorescein isothiocyanate-sinistrin (FITC-sinistrin) obtainable by a method as claimed in one of the claims 1 to 4.

6. Use of fluorescein isothiocyanate-sinistrin (FITC-sinistrin) as claimed in claim 5 as a component of a diagnostic preparation for renal diagnostics.

7. Diagnostic preparation containing fluorescein isothiocyanate-sinistrin (FITC-sinistrin).

8. Non-invasive method for measuring renal function comprising beaming light onto the skin of an individual to be examined and detecting the fluorescent light from the skin which is generated by fluorescein isothiocyanate-sinistrin (FITC-sinistrin) as claimed in claim 5 or by the diagnostic preparation as claimed in claim 7.

## Revendications

1. Procédé pour la préparation de sinistrine d'isothiocyanate de fluorescéine (FITC-sinistrine), dans lequel
i) on fait d'abord réagir la sinistrine dans un solvant approprié avec de l'hydrure de sodium,
ii) on ajoute ensuite du FITC au mélange réactionnel, et
iii) on isole la FITC-sinistrine sous forme solide.

2. Procédé selon la revendication 1, dans lequel le solvant dans l'étape i) est du diméthylformamide anhydre.

3. Procédé selon la revendication 1 ou 2, dans lequel l'étape iii) comprend l'addition au mélange réactionnel d'une solution aqueuse de chlorure d'ammonium, l'extraction consécutive avec du diéthyléther et l'élimination du solvant.

4. Procédé selon la revendication 3, dans lequel
iv) le solide de l'étape iii) est purifié par recristallisation et/ou filtration du gel.

5. Sinistrine d'isothiocyanate de fluorescéine (FITC-sinistrine) pouvant être obtenue par un procédé selon l'une quelconque des revendications 1 à 4.

6. Utilisation de la sinistrine d'isothiocyanate de fluorescéine (FITC-sinistrine) selon la revendication 5 comme constituant d'une composition diagnostique pour le diagnostic rénal.

7. Composition diagnostique contenant de la sinistrine d'isothiocyanate de fluorescéine (FITC-sinistrine).

8. Procédé non invasif pour la mesure de la fonction rénale comprenant l'irradiation de lumière sur la peau d'un individu à analyser et la détection de la lumière de fluorescence de la peau qui est produite par la sinistrine d'isothiocyanate de fluorescéine (FITC-sinistrine) selon la revendication 5 ou la composition diagnostique selon la revendication 7.
